# EUROPEAN PATENT SPECIFICATION

(11) **EP 3 773 013 B1**
(45) Date of publication and mention of the grant of the patent: **30.04.2025**
(21) Application number: 18718945.1
(22) Date of filing: 27.03.2018
(51) Int. Cl.: A23L 33/155, A61K 45/00, A23L 33/17

(54) **INSULIN CONTROL IN OVERWEIGHT OR OBESE ADULT DURING LIFE TIME INTERVENTION**
INSULINKONTROLLE BEI ÜBERGEWICHTIGEN ODER ADIPÖSEN ERWACHSENEN WÄHREND EINER LEBENSLANGEN INTERVENTION
RÉGULATION DE L'INSULINE CHEZ L'ADULTE EN SURPOIDS OU OBÈSE DURANT UNE INTERVENTION À VIE

(43) Date of publication of application: 17.02.2021
(73) Proprietor: N.V. Nutricia, 2712 HM Zoetermeer (NL)
(72) Inventor: WOPEREIS, Suzan, 2595 DA The Hague (NL); PASMAN, Wilrike, 2595 DA The Hague (NL); VOGEL DE, Johan, 3584 CT Utrecht (NL); WEIJS, Peter, 1067 SM Amsterdam (NL); MEMELINK, Robert, 1067 SM Amsterdam (NL)
(74) Representative: Nederlandsch Octrooibureau
(86) International application number: PCT/NL2018/050186
(87) International publication number: WO 2019/190306

(56) References cited:
- WO-A1-2014/200332
- WO-A1-2016/020485
- WO-A1-2016/020487
- TEEGARDEN D ET AL: "Vitamin D: emerging new roles in insulin sensitivity", NUTRITION RESEARCH REVIEWS, CAMBRIDGE UNIVERSITY PRESS, CAMBRIDGE, GB, vol. 22, no. 1, 1 June 2009 (2009-06-01), pages 82 - 92, XP002750620, ISSN: 0954-4224, [retrieved on 20090625], DOI: 10.1017/S0954422409389301
- A. M. VERREIJEN ET AL: "A high whey protein-, leucine-, and vitamin D-enriched supplement preserves muscle mass during intentional weight loss in obese older adults: a double-blind randomized controlled trial", THE AMERICAN JOURNAL OF CLINICAL NUTRITION, vol. 101, no. 2, 1 February 2015 (2015-02-01), US, pages 279 - 286, XP055344319, ISSN: 0002-9165, DOI: 10.3945/ajcn.114.090290

## Description

The invention rests in the field of management of insulin resistance, particularly in obese or overweight adult, participating in a weight loss program.

### BACKGROUND OF THE INVENTION

Insulin resistance (IR) is a pathological condition in which cells fail to respond normally to the hormone insulin. The body produces insulin when glucose starts to be released into the bloodstream from the digestion of carbohydrates in the diet. Normally this insulin response triggers glucose being taken into body cells, to be used for energy, and inhibits the body from using fat for energy. The concentration of glucose in the blood decreases as a result, staying within the normal range of approximately 5 mmol/L (mM) even when a large amount of carbohydrates is consumed. However, when the body produces insulin under conditions of IR, the cells are resistant to the insulin and are unable to use it as effectively, leading to high blood sugar. Beta cells in the pancreas subsequently increase their production of insulin, further contributing to a high blood insulin level. This can contribute to the development of pre-diabetes followed by type 2 diabetes.

WO 2016/020485 describes a combination of zinc and vitamin D for use in improving insulin sensitivity, treating or preventing an impaired glucose tolerance, type II diabetes, gestational diabetes mellitus, or preventing a condition associated with any of these conditions in a subject. Teegaarden & Donkin, Vitamin D: emerging new roles in insulin sensitivity, Nutrition Research Review, 2009, 22, 82- 92, investigates the role between vitamin D status and insulin sensitivity in adults using data collated from studies in the literature.

The worldwide high prevalence of overweight and obesity within an aging population creates challenges for society and health care systems. For instance, in The Netherlands at present more than 40% of the over 65 year old population is overweight and more than 15% is obese. Overweight, obesity and aging are all associated with a higher risk of IR.

It is thus important to find a method to ameliorate insulin resistance or, in other words, how to increase insulin sensitivity, by improvement of the management of insulin sensitivity during aging and especially in conditions of overweight and obesity.

### SUMMARY OF THE INVENTION

Verreijen et al "A high whey protein-, leucine-, and vitamin D-enriched supplement preserves muscle mass during intentional weight loss in obese older adults: a double-blind randomized controlled trial' Am J Clin Nutr. 2015 Feb;101(2):279-86 have shown that a high-protein, leucine and vitamin D enriched oral nutritional supplement (ONS) preserves skeletal muscle mass compared to an iso-caloric control supplement in older obese adults participating in a calorie restriction diet and resistance exercise program during a 13-week randomised controlled study.

For the present invention, the inventors surprisingly found that said composition did not show a significant effect on glucose uptake, but synergistically decreased insulin resistance, increased insulin sensitivity and decreased fasting insulin levels in obese or overweight older adults during a lifestyle intervention. A hypocaloric diet in combination with resistance exercise resulted in significant weight loss, improved fasting glucose levels and improved plasma HbA1c. Surprisingly, addition of the composition according to the invention on top of lifestyle intervention and hypocaloric diet did not have any effects on plasma glucose levels but showed additional benefits by decreasing insulin resistance, increasing insulin sensitivity and decreasing fasting insulin levels. These findings are particularly of value for those subjects that suffer or are at risk of increased insulin resistance, impaired insulin sensitivity and increased fasting insulin levels.

The invention pertains to a nutritional product for therapeutic use in decreasing fasting insulin levels, increasing insulin sensitivity or decreasing insulin resistance, in older obese or overweight adults of at least 45 years of age preferably during a lifestyle intervention, such as participating in a weight loss program involving a physical exercise regimen and a hypo-caloric dietary regimen, wherein said product comprises per serving between 50 - 300 kcal, preferably 100 - 200 kcal; between 10 g and 35 g proteinaceous matter, preferably 15- 25 g proteinaceous matter; and at least 2.5 microgram vitamin D. Preferably the serving is administered at least once per day as a single shot. Lifestyle intervention is preferably defined as any intervention including exercise, diet and/or a weight loss program. The lifestyle intervention is preferably a weight loss program involving a physical exercise regime and a hypocaloric dietary regime. The invention particularly pertains to obese or overweight adults of at least 45 years of age who are participating in a weight loss program, said older adult preferably following a hypocaloric diet in combination with an exercise regimen, preferably resistance exercise training. Preferably the serving is administered at least once per day as a single shot.

Preferably, the invention pertains to a nutritional composition or product with the above characteristics, in conjunction with a weight loss program for therapeutic use in decreasing fasting insulin levels, increasing insulin sensitivity or decreasing insulin resistance, in older obese or overweight adults of at least 45 years of age. Preferably the composition is administered at least once a day, in a single shot, to adults that suffer from overweight or obesity and/or adults suffering from a disease or condition selected from the group consisting of pre-diabetes type 2, or diabetes type 2.

In a preferred embodiment, said adults are fed 25 - 100 g dry weight of the nutritional composition daily, in a single dose or as multiple doses, wherein the nutritional composition has an energy density of between 200 and 500 kcal per 100 g dry weight, and, in terms of dry weight: between 45 and 55 wt% proteinaceous matter, including between 0.5 and 4 wt% leucine as free amino acid, peptide and/or salt thereof, less than 50wt% carbohydrates, and between 2 and 15 wt% dietary fiber, between 5 and 75 microgram vitamin D. Preferably the serving is administered at least once per day as a single shot.

The older obese or overweight adult is preferably suffering from a disease or condition selected from the group consisting of pre-diabetes type 2, or diabetes type 2.

### LIST OF FIGURES

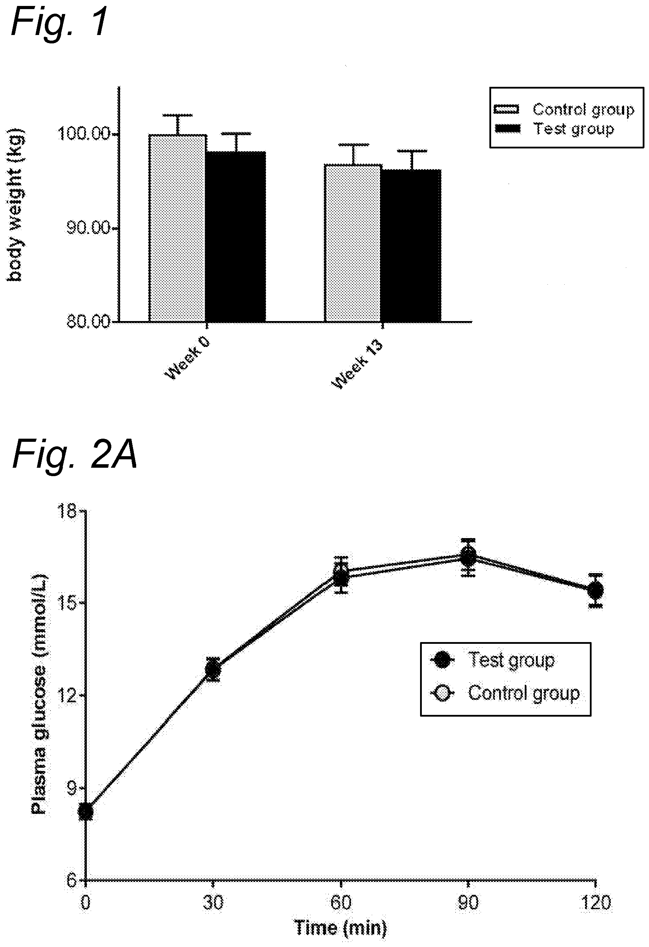

**Figure** 1: Weight loss in both the control and test group, at week 0 and week 13. Within the control group, from week 0 to week 13: p < 0.001. Within the test group, from week 0 to week 13: p < 0.001. The intervention effect was statistically significant compared to the change in the control group: p = 0.237, based on a linear mixed model including the baseline value in the outcome vector adjusting for stratification factors (gender and SU-derivate).

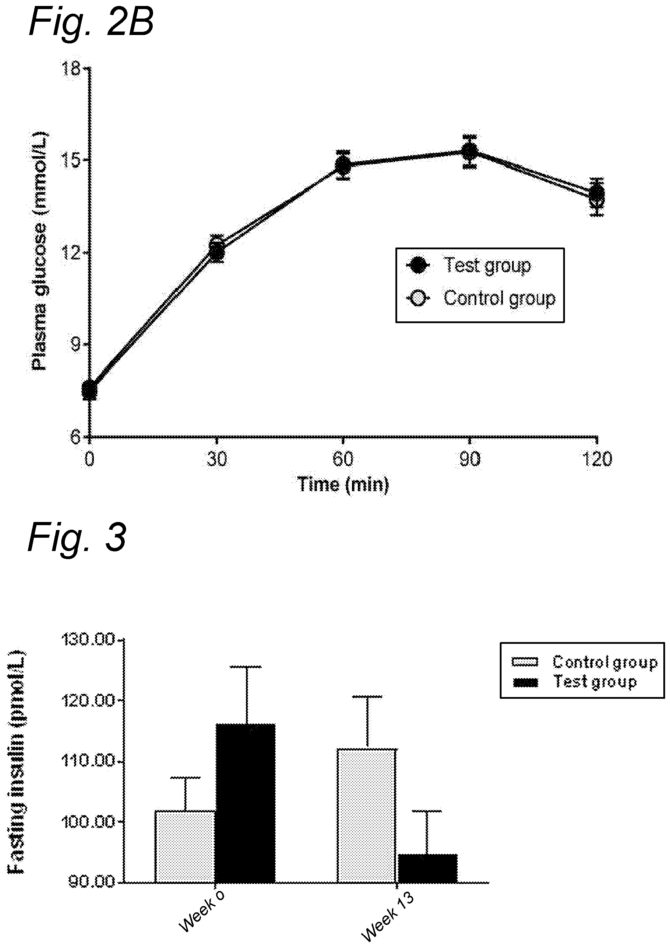

**Figure 2****:** Glucose concentrations at the individual time points of the oral glucose tolerance test, with the control and test groups compared at week 0 (Figure 2A) and at week 13 (Figure 2B).
**Figure 3****:** Fasting insulin concentrations at week 0 and 13. The data are means. From week 0 to week 13, within the control group: p = 0.147; within test (intervention) group: p = 0.003. The intervention effect was statistically significant compared to the change in the control group: p = 0.002, based on a linear mixed model including the baseline value in the outcome vector adjusting for stratification factors (gender and SU-derivate).

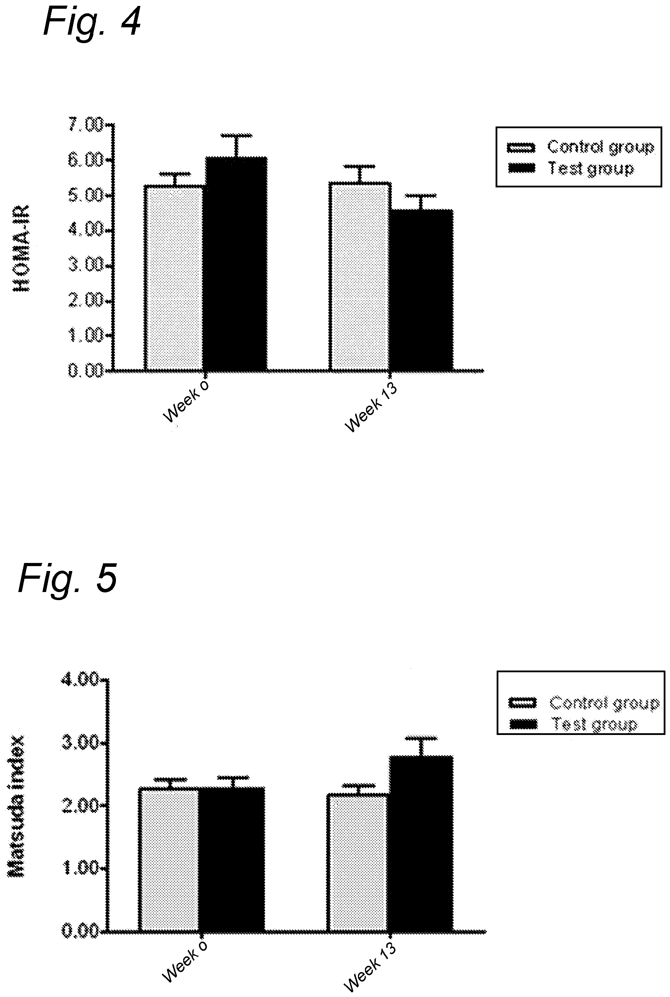

**Figure 4****:** Effect on HOMA-IR due to intervention at week 0 and week 13. The data are means. the intervention effect was statistically significant compared to the change in the control group: p = 0.011, based on a linear mixed model including the baseline value in the outcome vector adjusting for stratification factors (gender and SU-derivate).
**Figure 5****:** Effect on Matsuda index due to intervention at week 0 and week 13. The data are means. The intervention effect was statistically significant compared to the change in the control group: p = 0.018, based on a linear mixed model including the baseline value in the outcome vector adjusting for stratification factors (gender and SU-derivate).

### DETAILED DESCRIPTION OF THE INVENTION

Throughout the specification and claims, the older adult is a human being.

Obesity is defined as an unhealthy excess of body fat, which increases the risk of medical illness and premature mortality. BMI, calculated as body weight (in kg) divided by the square of height (in m), has been widely used and accepted as a simple method to classify medical risk by weight status. The classification 'overweight' is reserved for BMI of 25 - 30, while 'obesity' is characterized by a BMI of 30 or higher. The prevalence of obesity (defined as BMI of 30 or higher) among older adult is rising in The Netherlands. At present, more than 15% of the population above 65 years of age is obese. As the prevalence of both ageing and obesity is increasing, obese older adult are a significant target population for research. Obesity is clearly related to metabolic risk factors for cardiovascular diseases and diabetes mellitus.

Prediabetes is the precursor stage before diabetes mellitus in which not all of the symptoms required to diagnose diabetes are present, but blood sugar is abnormally high. Adults suffering from prediabetes are at increased risk for diabetes and cardiovascular disease (CVD). Prediabetes is associated with obesity. It is thus a metabolic diathesis or syndrome, and it usually involves no symptoms but impaired fasting blood sugar and impaired glucose tolerance which are similar in clinical definitions for diabetes.

The inventors surprisingly found that a nutritional supplement, specifically designed for older adults is particularly useful in decreasing fasting insulin levels, increasing insulin sensitivity and decreasing insulin resistance in older adults belonging to the group of pre-diabetes type 2 or diabetes type 2 during a weight loss program involving exercise or training. The results are reported in the clinical evidence further below.

In the context of this application, the term "at least" also includes the starting point of the open range. For example, an amount of "at least 95 wt%" means any amount equal to 95 weight % or above.

The term 'proteinaceous matter' comprises all protein, in intact or hydrolyzed form, di- and tripeptides as well as free amino acids, and salts thereof. It includes a protein or any part derivable of a protein, such as but not limited to non-hydrolyzed protein, native protein, hydrolyzed protein, peptides, such as oligopeptides and dipeptides, and amino acids. Leucine as non-protein bound free amino acid is part of proteinaceous matter, but for instance citrulline, and creatine would not fall within the definition.

In the context of this application, the older adult is an older adult human of the age of 45 years or more, more preferably of 50 years or more, in particular of the age of 55 or more, more in particular of the age of 60 or more.

In the context of this application the term "daily dose" means the serving size indicating the amount of the nutrition information which is administered to the older adult per day. Preferably a daily dose is limited to between 50 and 600 kcal, even more preferably between 100 and 300 kcal per day. Preferably the daily dose is given in 1 to 4 servings per day, more preferably 1 or 2 servings, or most preferably 1 serving per day.

It is preferred that the nutritional composition of the invention comprises predominantly fast-digestible protein (referring to the rate of appearance in the circulation of the amino acids following whey ingestion) and to consume the nutritional composition at least once in a single shot per day. With 'fast-digestible protein' it is understood to include hydrolyzates of any protein source (including vegetable proteins and milk protein sources), free amino acids, and/or whey protein. Since the taste of whey protein is most appreciated, this protein source is preferred. As further detailed below, it is preferred that the composition is leucine-enriched.

In the context of this application the term serving or administration at once means that the serving is preferably consumed as a shot within a time span of 15 minutes, more preferably within 10 minutes, even more preferably within 5 minutes.

In the context of the invention, with a 'weight loss program' it is understood a program involving (a) a dietary calorie restriction (i.e. a hypocaloric dietary regimen) in conjunction with (b) a physical exercise regimen.

The older adults are advised to adhere to a protocol of dietary restriction in terms of limited daily caloric intake, i.e. a 'hypo-caloric' or low-caloric dietary regimen. According to dietary guidelines, such a hypo-caloric dietary regimen preferably involves a restriction of the total daily caloric intake of a human being that is 10 - 50% of his or her average total daily intake. The average total daily intake of the target group of older adults is well-documented, and it is considered within the ambits of the skilled person's knowledge to determine a suitable meal plan and the caloric restrictions of a hypocaloric dietary regime. According to FAO publication; Human energy requirements, Report of a Joint FAO/WHO/UNU Expert Consultation Rome, 17-24 October 2001 the energy requirements of humans have been established. As summarised in the below table, cited from this report, the energy requirements depend on the age, bodyweight (BMI), and physical activity level (PAL). The content of the above report is herewith considered incorporated by reference.

According to the present invention the term "hypocaloric diet" preferably means a diet that delivers the daily energy expenditure (total energy expenditure; TEE) or less (in calories) when calculated without the physical exercise program. TEE may also be referred to in the art as the 'TDEE' (i.e. total daily energy expenditure). For instance, an overweight male between 40 and 50 years old has a TEE of 37 kcal/kg body weight. According to the invention, a hypo-caloric diet will thus provide at most 37 kcal/kg body weight. Dependent on patient's age and gender, the skilled person can straightforwardly calculate the caloric consequences of a hypo-caloric diet for an overweight or obese person from these tables. It is preferred that the hypo-caloric diet provides 25 - 95 %, preferably 60 - 90 %, more preferably 70- 90 % of the TEE of the targeted subject. Additionally or alternatively, in terms of absolute numbers the hypo-caloric diet may involve a caloric content of more than 500 kcal below estimated needs of the targeted subject, preferably assessed in terms of TEE or in accordance with WHO guidelines, more preferably a hypo-caloric diet of 600 - 1000 kcal below estimated needs of the targeted subject (preferably assessed through TEE or WHO guidelines), dependent on the subjects age, gender, BMI and daily activities. TEE may for instance be assessed by means of the doubly labelled water method (TEEDLW). This method is widely regarded as the 'gold standard' for estimating TEE in free-living individuals. Reference is made to for instance Melanson et al. "Physical activity assessment: a review of methods" Crit Rev Food Sci Nutr 1996; 36: 385 - 396, its contents herein incorporated by reference. TEE can also be estimated by methods such as indirect calorimetry of formulas such as the Harrison and Benedict multiplied with a physical activity score.

### Daily energy expenditure, basal metabolic rate and physical activity level measured in United States adults

| **Age years** | **No.** | **Weight kg** | **TEE measured with DLW** | | | | **BMR measured individually** | | | | **PAL** |
|---|---|---|---|---|---|---|---|---|---|---|---|
| | | | MJ | kJ/kg | *kcal* | *kcal*/*kg* | MJ | kJ/kg | *kcal* | *kcal*/*kg* | |
| **Overweight men** | | | | | | | | | | | |
| 20-30 | 10 | 89.9 | 13.5 | 150 | *3 224* | 36 | 7.8 | 86 | *1 858* | *21* | 1.90 |
| 30-40 | 53 | 102.4 | 15.5 | 151 | *3 703* | 36 | 8.6 | 84 | *2 046* | *20* | 1.81 |
| 40-50 | 37 | 94.6 | 14.5 | 153 | *3 465* | *37* | 7.9 | 83 | *1 878* | *20* | 1.88 |
| 50-60 | 17 | 100.3 | 14.5 | 144 | *3 458* | *34* | 7.8 | 77 | *1 857* | *19* | 1.88 |
| 60-70 | 30 | 87.8 | 11.9 | 136 | *2 851* | *32* | 7.1 | 80 | *1 687* | *19* | 1.71 |
| 70-80 | 34 | 84.8 | 11.0 | 129 | *2 624* | *31* | 7.2 | 85 | *1 713* | *20* | 1.55 |
| 80-90 | 7 | 78.1 | 9.6 | 123 | *2 294* | *29* | 6.5 | 83 | *1 558* | *20* | 1.47 |
| >90 | 2 | 77.5 | 7.8 | 101 | *1 863* | *24* | 6.5 | 84 | *1 550* | *20* | 1.29 |

| **Overweight women** | | | | | | | | | | | |
|---|---|---|---|---|---|---|---|---|---|---|---|
| 20-30 | 33 | 83.4 | 11.4 | 136 | *2 713* | *33* | 6.4 | 77 | *1 536* | *18* | 1.78 |
| 30-40 | 41 | 83.9 | 11.7 | 139 | *2 794* | *33* | 6.6 | 79 | *1 587* | *19* | 1.78 |
| 40-50 | 14 | 96.9 | 12.7 | 131 | *3 032* | *31* | 7.1 | 73 | *1 696* | *18* | 1.80 |
| 50-60 | 29 | 83.3 | 9.8 | 118 | *2 349* | *28* | 5.9 | 71 | *1 409* | *17* | 1.68 |
| 60-70 | 46 | 78.2 | 8.6 | 110 | *2 061* | 26 | 5.7 | 74 | *1 374* | *18* | 1.52 |
| 70-80 | 19 | 69.3 | 7.8 | 113 | *1 868* | 27 | 5.2 | 75 | *1 234* | *18* | 1.51 |
| 80-90 | 6 | 62.8 | 7.3 | 116 | *1 748* | 28 | 5.2 | 82 | *1 233* | *20* | 1.42 |
| >90 | 7 | 74.8 | 7.4 | 99 | *1 766* | 24 | 5.6 | 75 | *1 332* | *18* | 1.33 |

The physical exercise regimen involves any physical exercise or activity other than or in addition to daily living activities that contributes to a negative energy balance or an activity that costs calories during that activity. Physical activity examples can be, but not limited to, resistance exercise, aerobic exercise or flexibility training or combinations thereof. Endurance training is not recommended, at least not as the sole source of physical exercise It is preferred that the physical exercise regimen involves at least resistance exercise, due to its well-known effects on muscle mass maintenance and stimulation of muscle mass increase. Activity patterns could vary from engaging in the above one or more physical activities for a minimum of 1 time per week but preferably 2 times per week and more preferably 3 or more times per week, including at least some form of resistance exercise. There are ACSM/AHA Guidelines for flexibility, endurance and resistance exercise which can be of help to the skilled person to determine a suitable physical exercise regimen, and these are considered incorporated by reference here. The physical exercise regimen involves daily physical exercise activities as described above, preferably comprising resistance exercise. The physical exercise regimen is preferably in accordance with ACSM/AHA guidelines, their contents herein incorporated by reference. In a preferred embodiment, the physical exercise regimen involves resistance exercise according to ACSM/AHA Guidelines.

Depending on the design of the hypocaloric diet, within the weight loss program, the composition for use according to the invention preferably has a low caloric content, i.e. preferably not exceeding 150 kcal/100ml (i.e. at most 1.5 kcal per ml) or preferably not exceeding 120 kcal/100 ml (i.e. at most 1.2 kcal per ml). In an embodiment, the composition preferably has per serving a caloric content between 75-300 kcal preferably between 100 and 250 kcal, particularly 100 - 200 kcal of the composition administered to the older adult. In one embodiment, the composition has a caloric content of at most 300, preferably between 50 and 250 kcal fed to the older adult per day. In one embodiment, the composition has a caloric content of at most 250, preferably between 70 and 200 kcal per daily dose. A serving preferably involves 100 - 200 ml, more preferably 125 - 175 ml; and./or 20 - 45 g dry matter, more preferably 25 - 40 g dry matter. Unless specified otherwise, as a guide the skilled person could convert the relative amounts of the various ingredients in terms of caloric content to volume assuming a serving of 150 kcal and 150 ml.

An embodiment of the invention thus pertains to a nutritional composition for therapeutic use in decreasing fasting insulin levels, increasing insulin sensitivity or decreasing insulin resistance, in older obese or overweight adults of at least 45 years of age participating in a weight loss program, said composition comprising per daily dose between 10g and 35 g proteinaceous matter, at least 2.5 microgram vitamin D and between 50 - 300 kcal energy content, wherein preferably at least 13 wt%, more preferably between 13 and 20 wt% of the total proteinaceous matter is leucine (as the sum of all leucine bound or in free form). The proteinaceous matter preferably comprises at least 50 wt%, more preferably at least 75 wt% whey protein, based on proteinaceous matter.

An embodiment of the invention thus pertains to a nutritional composition for use in decreasing fasting insulin levels, increasing insulin sensitivity or decreasing insulin resistance, in older obese or overweight adults of at least 45 years of age participating in a weight loss program, comprising per serving between 50 - 300 kcal; between 10 g and 35g proteinaceous matter; and at least 2.5 microgram vitamin D, wherein the proteinaceous matter preferably comprises at least 50 wt%, more preferably at least 75% whey protein (based on proteinaceous matter) and wherein preferably at least 13 wt%, more preferably between 13 and 20 wt% of the total proteinaceous matter is leucine (as the sum of all leucine bound or in free form).

The preferred proteinaceous matter is detailed further on.

### Daily micro-nutritional requirements

Since food intake is reduced during a weight loss program the composition according to the invention is preferably enriched with nutritional ingredients like vitamins and minerals. In a preferred embodiment according to the invention the composition used in the weight loss program comprises all nutritional ingredients recommended according to the nutritional guidelines for the older adults including calcium source and vitamin D source. Preferably a serving of the composition for use according to the invention comprises calcium in an amount between 150 and 1000 mg. A serving of the composition for use according to the invention comprises at least 2.5 microgram vit D, preferably between 2.5 and 60 microgram and even more preferably between 5 and 50 microgram vit D (per serving). The RDA [recommended daily allowance] of vitamin D is about 15 microgram on basis of low sun exposure. Due to low sun light exposure of many older adults the inventors believe that high doses of vit D are particularly useful in the older adult target group. A serving for use according to the invention comprises at least 2.5, more preferably at least 5 and even more preferably at least 10 microgram vitamin D per 100kcal of the serving. The term 'vitamin D' in the context of the invention refers to all physiological forms of vitamin D and its metabolite (i.e. 25 OHD), either D₁, D₂, D₃ or D₄, in particular D₂ and D₃, or any mixture thereof. In the context of this application, 1 IU of vitamin D is the biological equivalent of 0.025 µg. Hence, 1,000 IU is the biological equivalent of 25 µg.

### Proteinaceous matter

The protein or proteinaceous content of the composition comprises a sufficient amount of protein to assure at least a protein intake of between 10 and 35 g protein, preferably between 12 and 35 and even more preferably between 15 and 30 g protein per serving. In the preferred embodiment the serving(s) are consumed at or around breakfast, since most breakfasts are low in protein, or around the moment of exercise. Alternatively, in a preferred embodiment a daily protein intake as supplemented to the older adults provides at least 0.1 g proteinaceous matter / kg body weight, preferably at least 0.15, even more preferably more than 0.2 and most preferably at least 0.25 g proteinaceous matter per kg body weight. In a most preferred embodiment, there is less than 0.40 g proteinaceous matter per kg body weight provided.

The source of proteinaceous matter may be provided separately, in the form of a meal, a food supplement, a drink, or in any other form or may be combined in a single nutritional composition.

The proteinaceous matter originates from high quality protein, preferably high in branched chain amino acids. Proteins such as vegetable proteins soy or pea, more preferably animal protein such as egg or dairy, even more preferably fast digestible animal protein, such as whey protein are used. The amino acids are essentially L-amino acids as only L-amino acids are metabolically relevant in the context of this invention. The protein fraction is preferably high in branched chain amino acids (leucine, valine, isoleucine). An advantage of providing these amino acids in the form of protein is to avoid the adverse sensory impact of added amino acids, but also to create a favourable physiological response due to the high protein composition.

The nutritional composition according to the invention comprises 10 - 35 g of proteinaceous matter per serving. Preferably, the composition comprises at least about 12 g, at least about 15 g, and most preferably at least about 20 g of proteinaceous matter per serving. In a most preferred embodiment, there is less than 25 g proteinaceous matter per serving.

According to another embodiment, the nutritional composition according to the invention comprises at least about 45 en% of proteinaceous matter per 100 kcal. Preferably, the composition comprises between 35 and 80 en% of proteinaceous matter per 100 kcal. Preferably at least about 35, 40, or 48 en%, at least about 50 en%, at least about 52 en%, at least about 54 en%, and most preferably at least about 55 en% of proteinaceous matter per 100 kcal.

According to a preferred embodiment, the proteinaceous matter according to the invention comprises between 50 and 95 wt% whey protein, preferably at least about 80 wt% of whey protein, preferably at least about 85 wt% of whey protein, preferably at least about 90 wt%, and most preferably about 95 wt% of whey protein. Whey protein is considered a fast protein referring to the rate of appearance in the circulation of the amino acids following whey ingestion. In addition, whey protein is inherently high in branched chain amino acids (leucine, valine, isoleucine).

The whey protein may be provided as an intact whey protein, a hydrolysed whey protein, a microparticular whey protein, a nanoparticular whey protein, a micellar whey protein, and the like. Preferably, the whey protein is an intact whey protein, i.e. a whey protein in its intact form, such as present in fresh milk. As a source of whey protein to be used in the present invention, any commercially available whey protein source may be used, i.e. whey obtained by any process for the preparation of whey known in the art, as well as whey protein fractions prepared thereof, or in the form of proteins that constitute the bulk of the whey, i.e. being β-lactoglobulin, α-lactalbumin and serum albumin. The whey protein may be provided as liquid whey, or whey in powder form, such as whey protein isolate (WPI) or whey protein concentrate (WPC). Whey protein concentrate is rich in whey proteins, but also contains other components such as fat, lactose and glycomacroprotein (GMP), a casein-related non-globular protein. Typically, whey protein concentrate is produced by membrane filtration. On the other hand, whey protein isolate consists primarily of whey proteins with minimal amounts of fat and lactose. Whey protein isolate usually requires a more rigorous separation process such as a combination of microfiltration and ultra-filtration or ion exchange chromatography. It is generally understood that a whey protein isolate refers to a mixture in which at least 90 wt% of the solids are whey proteins. A whey protein concentrate is understood as having a percentage of whey proteins between the initial amount in the by-product (about 12 wt%) and a whey protein isolate. In particular, sweet whey, obtained as a by-product in the manufacturing of cheese, acid whey, obtained as a by-product in the manufacturing of acid casein, native whey, obtained by milk microfiltration or rennet whey, obtained as a by-product in the manufacturing of rennet casein, may be used as a source of whey proteins.

Furthermore, whey proteins may originate from all kinds of mammalian animal species, such as, for instance cows, sheep, goats, horses, buffalo's, and camels. Preferably, the whey protein is of bovine origin.

Preferably, the whey protein source is available as a powder, preferably the whey protein source is a WPC or WPI.

According to another embodiment, the proteinaceous matter according to the invention comprises at least about 45 wt% of essential amino acids (EAA), preferably at least about 47 wt%, and more preferably at least about 50 wt% of EAA. Essential amino acids are amino acids selected from the group of isoleucine (Ile), leucine (Leu), lysine (Lys), methionine (Met), phenylalanine (Phe), threonine (Thr), tryptophan (Trp), histidine (His) and valine (Val). Since native whey protein and casein protein comprise (depending on the source) maximum about 45 and 41 wt% of EAA, respectively, it may be necessary to add EAAs to the nutritional composition, such as in the form of amino acids or peptides, to arrive at the preferred amount of at least 45 wt%.

According to another embodiment, the proteinaceous matter according to the invention comprises total leucine, total valine and total isoleucine in a total leucine:valine:isoleucine weight ratio of about 1.7-3:1:1, preferably about 2:1:1. Alternatively, the weight ratio of leucine : (valine + isoleucine) is about 0.9 or higher, preferably 1.0 or higher. Suitable valine and isoleucine levels may be provided by the whey protein, or may be provided by added amino acids, either in free form as bases or salts, or as peptides.

### Anabolic amino acid

The composition of the present invention preferably involves the use of an anabolic amino acid-derived stimulus, which is defined as a chemical compound derived from or which is a precursor to an amino acid (hence, amino acid derived).

Preferably, the anabolic amino acid-derived stimulus is one or more selected from the group of L-leucine, β-hydroxy-β-methylbutyrate, β-hydroxy-β-methylbutyrate free acid, calcium-β-hydroxy-β-methylbutyrate, hydroxyl iso caproic acid, ketoisocaproic acid, citrulline, and creatine. Most preferably, the anabolic amino acid-derived stimulus is an amino acid selected from the group of L-leucine, β-hydroxy-β-methylbutyrate and citrulline. β-hydroxy-β-methylbutyrate and citruline have an improved effect on the palatability and hence compliance of the product compared to L-leucine. It is particularly preferred to use β-hydroxy-β-methylbutyrate or citrulline or both in concentrations higher than 1 g/100 ml. L-leucine (hereafter also called leucine, since the R-form of leucine is biologically not relevant in the context of this invention) is an essential amino acid, being part of a diverse number of proteins and, together with valine and isoleucine, belongs to the group of branched-chain amino acids. Leucine may be used as a free amino acid, or in a bound form, such as a dipeptide, an oligopeptide, a polypeptide or as part of a protein. Common protein sources of leucine are dairy proteins such as whey, casein, micellar casein, caseinate, and glycomacroprotein (GMP), and vegetable proteins such as wheat, rice, pea, lupine and soy proteins. Said sources of protein may provide intact proteins, hydrolysates or mixtures thereof, hereafter further called proteinaceous matter.

In one embodiment, anabolic amino acid is provided in a daily dosage of 1 to 10 g. In one embodiment, it is preferred to provide at least leucine. When leucine is provided as anabolic amino acid, the proteinaceous matter preferably comprises at least about 13 wt% of leucine, based on the total weight of the proteinaceous matter. Preferably, said proteinaceous matter comprises between 13 and 20 wt% of leucine. In a preferred embodiment part of the leucine may be replaced by one or more amino acid derived stimuli mentioned above, preferably anabolic amino acids selected from the group consisting of β-hydroxy-β-methylbutyrate, β-hydroxy-β-methylbutyrate free acid, calcium-β-hydroxy-β-methylbutyrate, hydroxyl iso caproic acid and ketoisocaproic acid, creatine and citrulline.

Preferably, at least about 20 %, preferably at least about 22.5 %, preferably at least about 25 % of total leucine is provided in free form, relative to the total amount of leucine. In the context of this application, with "free form" includes a peptide comprising 1 to 5 amino acids, preferably 1 to 3 amino acids, more preferably 1 amino acid. Preferably, leucine is a free amino acid, either as a base, a salt or a chelate. It is preferred that at most 40 %, more preferably at most 35 % of the leucine is provided as in free form, most preferably as a free amino acid.

Citrulline is an α-amino acid. Citrulline, in the form of citrulline malate, is sold as a performance-enhancing athletic dietary supplement which was suggested to promote aerobic energy production (human study) and to increase athletic performance and decreasing muscle soreness (human study). In the human body, citrulline is produced from ornithine and carbamoyl phosphate in one of the central reactions in the urea cycle. It is also produced from arginine in the body as a by-product of the reaction catalyzed by NOS family. Citrulline is also capable of promoting muscle protein synthesis and has been described in human and animal studies [see e.g. WO 2008/049984 by Université René Descartes-Paris, 2 May 2008]. Citrulline is commercially available and can be obtained, e.g. from Ajinomoto, Kyowa, and Biocodex. In one embodiment, citrulline is provided in a daily dosage between 0.5 to 10 g, preferably between 0.8 and 8, and even more preferably between 1 and 5 g.

Creatine (N-(amino-imino-methyl)-N-methyl-glycine; methylglycocyamine) is a nitrogenous organic acid that is produced in vertebrates, in particular the human body from L-arginine, glycine, and L-methionine and helps to supply energy to muscles. Creatine is commercially available and can be obtained, e.g. from Sigma Aldrich, Alfa Aesar, and Aminolabs. In one embodiment, creatine is provided in a daily dosage of 0.5 to 20 g, preferably between 1-15, even more preferably between 1.5 and 10 g.

Preferably, the anabolic amino acid-derived stimulus is provided in a daily dosage of 0.5 to 20 g, preferably 1 to 10 g. Preferably, such daily dosage is administered as a single serving.

In one embodiment, any combination of leucine, β-hydroxy-β-methylbutyrate and citrulline, citrulline, and creatine is provided in a daily dosage of 0.5 to 20 g, preferably 1 to 10 g.

According to one embodiment, the anabolic amino acid-derived stimulus in combination with vitamin D is used for the manufacture of a medicament. In the context of this application, a medicament is an embodiment of the invention which does not, or does not substantially contain caloric components, such as carbohydrates, fat and proteinaceous matter, other than the components according to the invention.

The medicament may be administered sequentially or simultaneously with said caloric components, in particular a protein source. Said caloric components may be provided separately, in the form of a meal, a food supplement, a drink, or in any other form.

According to an alternative embodiment, the anabolic amino acid-derived stimulus in combination with vitamin D is used for the manufacture of a nutritional composition. In the context of this application, a nutritional composition is an embodiment of the invention which contains, or substantially contains caloric components, such as carbohydrates, fat and proteinaceous matter, other than the components according to the invention.

According to one embodiment, the anabolic amino acid-derived stimulus in combination with vitamin D is used in combination with a source of proteinaceous matter to provide the necessary amino acids to prevent and/or treat a loss of muscle mass, a loss of muscle function, or both, in the older adult targeted.

### Fat and carbohydrates

According to a preferred embodiment, the nutritional composition according to the invention comprises at least one of a source of fat and a source of carbohydrates. The presence of one or both of these components effectively prohibits the excessive use of the protein as an energy source instead. Preferably the composition according to the invention comprises at least 20 en% carbohydrates and/or fat.

The total amount of energy supplied by the fat and/or carbohydrates (digestible and indigestible) preferably match the total energy supplied by the proteinaceous matter. Therefore, the total amount fat and/or carbohydrates preferably is at most about 55 en%, preferably at most about 52 en %, preferably at most about 48 en%, preferably at most about 43 en%.

In a preferred embodiment, the nutritional composition according to the invention comprises a source of fat and a source of carbohydrates, preferably in an amount of about 2 g of fat and about 6 g of digestible carbohydrates per 100 kcal.

With regard to the type of fat, a wide choice is possible, provided the fat is of food quality. The fat may either be an animal fat or a vegetable fat or both. Although animal fats such as lard or butter have essentially equal caloric and nutritional values and can be used interchangeably, vegetable or marine oils are highly preferred in the practice of the present invention due to their readily availability, absence of cholesterol and lower concentration of saturated fatty acids. The fat may include a source of medium chain fatty acids, such as medium chain triglycerides (MCT, mainly 8 to 10 carbon atoms long), a source of long chain fatty acids, such as long chain triglycerides (LCT) and phospholipid-bound fatty acids such as phospholipid-bound EPA or DHA, or any combination of the two types of sources. Unsaturated preferably mono-unsaturated, most preferably mono and poly-unsaturated LCT sources, such as canola oil, rapeseed oil, sunflower oil, soybean oil, olive oil, coconut oil, palm oil, linseed oil, marine oil or corn oil are beneficial because it is known that these LCTs may have beneficial effects in the human body. In a preferred embodiment according to the invention, the composition comprises a source of long chain poly unsaturated fatty acids (IcPUFA). Preferably the PUFA comprises at least 30% alpha linolenic acid (C18, n3), eicosopentaenoic acid (EPA, C20, n3), or docosahexaenoic acid (C22, n3 DHA).

With regard to the type of carbohydrates, a wide choice is possible, provided the carbohydrates are of food quality. The digestible carbohydrates positively influence the energy level of a subject, and add to the advantageous effect of the nutritional composition according to the invention. The digestible carbohydrate may comprise either simple or complex carbohydrates, or any mixture thereof. Suitable for use in the present invention are glucose, fructose, sucrose, lactose, trehalose, palatinose, corn syrup, malt, maltose, isomaltose, partially hydrolysed corn starch, maltodextrins, glucose oligo- and poly-saccharides. More preferably sugars with a low glycemic index (GI) are used. Low GI sugars are sugars that are slowly released in the blood compared to glucose. Examples are fructose, palatinose, isomaltulose or maltodextrins. Preferably carbohydrates are used with a GI below 70.

### Dietary fibers

The composition for use according to the invention may optionally be fortified with dietary fibres or non-digestible carbohydrates such as galacto-oligosaccharides, fructooligosaccharides, inulin, and pectin (hydrolysed pectin, low-viscosity pectin (a pectin degradation product with a DP of 2 - 250), or other pectin degradation products). In an embodiment of the present invention, the composition according to the invention comprises 0.5 g/serving to 6 g/serving of non-digestible carbohydrates, preferably 1 g/serving to 1.5 g/serving. The dietary fibres include non-digestible oligosaccharides having a DP of 2 to 20, preferably 2 to 10. More preferably, these oligosaccharides do not contain substantial amounts (less than 5 weight %) of saccharides outside these DP ranges, and they are soluble.

Preferably, the nutritional composition further comprises one or more dietary fibres, preferably having a degree of polymerization [DP] of 2 to 250, more preferably a DP of 2 to 100, more preferably DP 2 - 50. The composition preferably comprises one or more dietary fibres selected from the group of short chain galactooligosaccharides (GOS), preferably having a DP 2 - 10, preferably DP 2 - 8, long chain fructooligosaccharides (FOS), including inulin (preferably having an average DP 8 - 50), and low-viscosity pectin (preferably having an average DP 2 - 50). The low-viscosity providing pectin such as Herbapekt SF 50-LV has a molecular weight of about 25,000 Dalton to ensure its solubility. Due to the low viscosity it can be used in very high dosage without having a major influence on the texture of the desired product which is a beneficial effect of this type of pectin. In one embodiment, the composition preferably comprises at least one fiber selected from the group consisting of GOS and FOS, more preferably a mixture of GOS and FOS.

### Medical use

The nutritional composition according to the invention can advantageously be used for the prevention or treatment of a disease or condition involving decreasing fasting insulin levels, increasing insulin sensitivity or decreasing insulin resistance, in an older adult as defined here above, preferably an older adult participating in a weight loss program. Alternatively, the nutritional composition according to invention can advantageously be used for the prevention or treatment of a disease or condition selected from the group of (pre)-diabetes type 2, or diabetes type 2, in an older adult, preferably an older adult participating in a weight loss program. The weight loss program preferably comprises resistance exercise. The composition is preferably part of a hypocaloric diet, wherein the older adult receives (i) at least 500 kcal, preferably 500 - 700 kcal less than the daily energy expenditure (TEE) of the older adult, or (ii) 25 - 95 %, preferably 60 - 90 %, more preferably 70- 90 % of the TEE of the older adult. The older adult preferably suffers from overweight or obesity.

### EXPERIMENTAL EVIDENCE

Clinical study shows decreased fasting insulin levels, increased insulin sensitivity and decreased insulin resistance during a weight loss program in older adult.

### Material and Methods:

### Subjects

Men and women (55 to 85 years old, inclusive) with obesity (BMI higher than 30kg/m², or when BMI higher than 27kg/m² with waist circumference higher than 88cm (women) and > 102cm (men)) being ambulant (pre-) type 2 diabetes patient were recruited from the Dutch population. Subjects were excluded from participation if they had had any malignant disease during the last five years, participated in any weight loss program three months before start of the study, it was potentially unsafe to participate in the resistance training program according to a physiotherapist, or if they were not able to comply fully with study protocol.

### Design and randomization procedure

A randomized, controlled, double-blinded, parallel-group, single-centre study design was applied. All subjects were enrolled in a 13-week hypo-caloric weight loss diet and participated in a resistance exercise program. Subjects were randomly assigned to receive one of the study products (test or control) and stratified for gender.

### Hypo-caloric diet

All subjects followed a hypo-caloric weight loss diet of 600 kcal below estimated needs per day. Subjects had to take the study product once or twice daily throughout the 13-week intervention period; once daily at the days when no training takes place and twice-daily at the training days. The study product was taken in the morning, just before breakfast. At the training days, the second serving was taken after the training. The product had to be consumed completely, preferably as a bolus within 5, maximally 10 minutes.

The test product involved a nutritional supplement comprising a whey protein and was enriched with leucine. Per serving it contained 21 g total protein of which 20 g whey protein, 3 g total leucine, 9 g carbohydrates, 1.25 g fibre and 3 g fat, a caloric content of about 150 kcal per serving. The control product contained no protein. Taste and appearance of the control product were similar to test product and both products provided an energetic value of 150 kcal per serving.

### Resistance exercise program

The resistance exercise program was performed 3 times per week for a period of 13 weeks. The training started with a short warm up and was followed by several arm, leg and core strength exercises. The number of repetitions and intensity of the training was adapted to the personal ability of the participant.

### Sample collection for laboratory measurements

At baseline and after 13 weeks, a 2-hour oral glucose tolerance test (OGTT) was conducted. Cannulas were placed at least 20 minutes before the first blood sample were taken. Subjects had to consume a sugar solution (75 g sugar dissolved in 300 mL) within 5 minutes. To determine plasma glucose and insulin, a venous blood sample was drawn immediately (t=0 min) prior to intake of the sugar solution and 30, 60, 90 and 120 minutes after intake of the sugar solution. HbA1c was also measured in baseline plasma from all subjects.

### Calculations of the indices

### a) Homeostatic model assessment- insulin resistance (HOMA-IR)

Homa-IR is method that quantifies a degree of basal insulin resistance in a subject. For the calculation the fasting glucose (FPG) and fasting insulin (FPI) values are used in the following formula: HOMA-IR = (FPI × FPG)/22.5 where FPI is fasting plasma insulin concentration (mU/I) and FPG is fasting plasma glucose (mmol/l).

### b) Matsuda index

The Matsuda index is an index that reflects whole-body insulin sensitivity as calculated from the plasma glucose and plasma insulin response during an oral glucose tolerance test (OGTT). The index is calculated according the following formula: (10,000/square root of [fasting glucose x fasting insulin] x [mean glucose x mean insulin during OGTT]). It has been shown that the calculation of the index has a high correlation with the rate of whole body glucose disposal during an hyper-insulinaemic euglycemic clamp (Matsuda M, DeFronzo RA : Insulin sensitivity indices obtained from oral glucose tolerance testing: comparison with the euglycemic insulin clamp. Diabetes Care 22:1462-1470, 1999.)

### Statistics

Outcome parameters were analysed using a repeated measures mixed model with a random effect for subjects and a fixed factor for study group, sex and use of SU-derivatives at study start (screening) yes/no.

The Intention-to-Treat dataset was described as the primary dataset in the protocol and the Statistical Analysis Plan.

### Subject characteristics

Subject characteristics are presented in Table 1 and showed no significant differences between groups.

**Table 1: Subject characteristics in means (sd) unless otherwise specified**

| **Statistics/ Parameter levels** | **Control (N=61)** | **Test (N=62)** |
|---|---|---|
| Age (y) | 65.82 (6.45) | 66.84 (6.03) |
| Female (n, % female) | 23 (37.70%) | 20 (32.26%) |
| Waist circumference (cm) | 115.9 (10.7) | 114.1 (9.4) |
| Body weight (kg) | 101.49 (15.78) | 99.69 (15.37) |
| BMI (kg/m2) | 33.99 (4.59) | 33.29 (4.32) |
| Duration type 2 diabetes (months from diagnosis) | 78.43 (57.28) | 93.66 (83.24) |
| Fasting glucose at V1a (mmol/L) | 8.24 (1.90) | 8.25 (1.76) |
| HbA1c (mmol/mol) | 53.0 (10.9) | 51.1 (9.7) |
| Use of SU-derivatives at screening | 23 (37.7%) | 21 (33.9%) |
| Use of metformin at screening | 52 (85.2%) | 48 (77.4) |

### Surprising effect on decreasing fasting insulin levels, increasing insulin sensitivity and decreasing insulin resistance after 13 weeks of intervention

AS shown in Fig 1, both the control group and the test group loss weight during the intervention.

The glucose concentrations at the individual time points of the oral glucose tolerance test, both at week 0 and week 13, are presented below, and visualized in **Figures 2A** and **2B****,** respectively. No statistically significant differences between groups were observed for the effect on the concentration of fasting glucose **(Table 2)** or HbA1c **(Table 3)** and the area under the curves of both glucose as shown in said **Fig. 2****.**

**Table 2: change in fasting glucose (mmol/L)**

| | **Control** | **Test** | **Intervention effect** |
|---|---|---|---|
| **week 13 vs. week 0** | -0.7 (1.5) | -0.7 (1.8) | P=0.94 |
| **(standard deviation)** | P=0.005 | P=0.004 | |

**Table 3: change in HbA1c (mmol/mol)**

| | **Control** | **Test** | **Intervention effect** |
|---|---|---|---|
| **week 13 vs. week 0** | -5.6 (8.7) | -4.2 (6.7) | P=0.39 |
| **(standard deviation)** | P<0.001 | P<0.001 | |

However, the fasting insulin concentration was decreased in the test group and increased in the control group, which was a statistically significantly difference between study groups **(Table 4).** The results are plotted in **Fig. 3****.**

**Table 4: Statistics of fasting insulin (pmol/L)**

| | **Control** | **Test** | **Intervention effect** |
|---|---|---|---|
| **week 13 vs. week 0** | 9.0 (45.2) | -19.7 (46.8) | P=0.002 |
| **(standard deviation)** | P=0.15 | P=0.003 | |

Further, there is an improvement on insulin sensitivity and a decrease in insulin resistance due to the intervention, as shown in **Fig 4** and **Fig. 5****,** respectively. The results are statistically significant.

### Examples of nutritional compositions:

According to one embodiment, the invention concerns a solid nutritional composition, suitable as a supplement within a calorie restriction protocol, comprising per 100 g of dry matter:
- About 378 kcal
- About 52 g of proteinaceous matter comprising about 90 weight % of whey protein, relative to the total proteinaceous matter, and which comprises about 13 weight % of leucine, relative to the total proteinaceous matter, of which about 25 weight % is in a free form, relative to the total leucine.
- About 7.5 g of fat of which 5.5 g unsaturated, about 3.2 g soluble fibre and about 24 g of carbohydrates
- About 50 µg Vit D3, 19 mg vitamin E, about 500 µg Folic Acid, about 7.5 µg Vitamin B12, about 1250 mg Calcium, about 630 mg Phosphorus, other vitamins and micronutrients at or below Food for special medical purposes (FSMP) regulation.

According to one embodiment the invention concerns a nutritional composition, suitable as a meal replacement in a caloric restriction setting during a weight loss program, comprising per serving:
- 200 kcal
- 40 energy % protein
- About 90% of the proteinaceous matter comprising of whey protein, with >13% leucine
- 6.5 g of fat, of which at least 1 g linoleic acid
- 16 g carbohydrates with 12 g as digestible carbohydrates and preferably more than 50% classified as low GI sugar, such as, but not limited to: fructose, isomaltulose, lactose, in addition 4 g of fibre, wherein the fibre is at least one selected form the group consisting of galactooligosaccharide (GOS), Fructooligoosaccharide (FOS), inulin and pectin.
- 800 IU Vitamin D, and other vitamins and micronutrients at or above EFSA regulation directive 96-8

According to a further embodiment the invention concerns a nutritional composition, suitable as snack replacer in a weight loss therapy, comprising per serving:
- 125 kcal
- 38-60 energy % protein, at least 90% of the proteinaceous matter comprising of whey protein and at least 13wt% leucine of the total proteinaceous matter
- 2 g fat, preferably more than 30% unsaturated
- 7 g of carbohydrates with a source of digestible carbohydrates containing preferably more than 50% of the digestible carbohydrate fraction consisting of low glycemic index sugars, such as, but not limited to, fructose, isomaltulose or lactose
- 2 g of fibre
- 2.5 µg or higher Vitamin D₃
- Optionally additional vitamins and minerals

According to a further embodiment the invention concerns a nutritional composition, suitable as a complete meal replacer during a weight loss comprising per serving:
- 300 kcal
- 35 energy % proteinaceous matter
- 80% of the proteinaceous matter comprising of whey protein, and supplemented with leucine to a total of at least 13wt% leucine of the total proteinaceous matter
- 10 g of fat, of which at least 50 wt% is unsaturated
- 30g carbohydrates with 25 g as digestible carbohydrates and preferably more than 50% classified as low GI sugar, such as, but not limited to: fructose, isomaltulose, lactose, in addition 5 g of fibre with preferably a mix of soluble and insoluble fibers
- 800 IU Vitamin D₃ and 1000 mg calcium
- Optionally additional vitamins and minerals.

## Claims

1. A nutritional product for use in
a) decreasing fasting insulin levels; or
b) increasing insulin sensitivity; or
c) decreasing insulin resistance
in obese or overweight adults of at least 45 years of age,
said product comprising per serving between 50 - 300 kcal; between 10 g and 35g proteinaceous matter; and at least 2.5 microgram vitamin D.

2. The product for use according to claim 1, wherein the adult is suffering from a disease or condition selected from the group consisting of pre-diabetes type 2 or diabetes type 2.

3. The product for use according to any one of the preceding claims, wherein the treatment comprises a daily dose of 1 to 4 servings.

4. The product for use according to any of the preceding claims, wherein the overweight or obese adults are participating in a weight loss program, wherein the weight loss program preferably comprises a hypo-caloric diet in combination with an exercise regimen.

5. The product for use according to claim 4, wherein said hypo-caloric diet involves a daily energy uptake of less than the daily energy expenditure (TEE) of the older adult, wherein the hypo-caloric diet preferably provides 25 - 95 %, preferably 60 - 90 %, more preferably 70- 90 % of the TEE of the obese or overweight adult.

6. The product for use according to any of claims 4 or 5, wherein said exercise regimen involves resistance exercise training.

7. The product for use according to any of the preceding claims, wherein the serving is administered as a single shot to be consumed within 15 minutes, more preferably within 10 minutes, even more preferably within 5 minutes.

8. The product for use according to any of the preceding claims, wherein at least 13wt%, preferably between 13 and 20 wt% of the total proteinaceous matter is leucine.

9. The product for use according to any one of the preceding claims, wherein the composition comprises per serving at least 0.5 gram of at least one compound selected from the group consisting of free leucine, salts of free leucine, or metabolites of leucine, or salts of metabolites of leucine, wherein the metabolite is preferably selected from the group consisting of β-hydroxy-β-methylbutyrate, β-hydroxy-β-methylbutyrate free acid, calcium-β-hydroxy-β-methylbutyrate, hydroxyl iso caproic acid and ketoisocaproic acid, and wherein the free leucine, salts thereof or its metabolites or the isoleucine or valine are preferably encapsulated.

10. The product for use according to any of the preceding claims, wherein the composition further comprises at least 20 en% carbohydrates and/or fat.

11. The product for use according to claim 10, wherein the carbohydrates comprise at least 10 wt%, based on total carbohydrate matter, of a carbohydrate with a glycemic index below 70.

12. The product for use according to claim 10, wherein the fat comprises at least 30 wt% of unsaturated fatty acids and at least 15 wt% of polyunsaturated acids of total fat in the composition.

13. The product for use according to any one of the preceding claims, wherein the proteinaceous matter comprises at least 60 wt% mammalian whey, preferably bovine whey.

14. The product for use according to any of the preceding claims wherein the serving comprises between 0.5 and 6 g dietary fiber, wherein the dietary fiber comprises at least one fiber selected from the group consisting of galactooligosaccharides (GOS), fructooligosaccharides (FOS), inulin and pectin oligosaccharides, preferably at least one fiber selected from the group consisting of GOS and FOS, more preferably a mixture of GOS and FOS.

## Patentansprüche

1. Ernährungsprodukt zur Anwendung bei
a) der Senkung des Nüchterninsulinspiegels; oder
b) der Erhöhung der Insulinempfindlichkeit oder
c) der Senkung der Insulinresistenz
bei adipösen oder übergewichtigen Erwachsenen von mindestens 45 Jahren,
wobei das Produkt pro Portion zwischen 50 und 300 kcal; zwischen 10 g und 35 g proteinhaltige Substanz; und mindestens 2,5 Mikrogramm Vitamin D umfasst.

2. Produkt zur Anwendung nach Anspruch 1, wobei der Erwachsene an einer Krankheit oder einem Zustand leidet, die bzw. der aus der Gruppe bestehend aus Prädiabetes Typ 2 oder Diabetes Typ 2 ausgewählt ist.

3. Produkt zur Anwendung nach einem der voranstehenden Ansprüche, wobei die Behandlung eine tägliche Dosis von 1 bis 4 Portionen umfasst.

4. Produkt zur Anwendung nach einem der voranstehenden Ansprüche, wobei die übergewichtigen oder adipösen Erwachsenen an einem Gewichtsabnahmeprogramm teilnehmen, wobei das Gewichtsabnahmeprogramm vorzugsweise eine hypokalorische Diät in Kombination mit einem Trainingsprogramm umfasst.

5. Produkt zur Anwendung nach Anspruch 4, wobei die hypokalorische Diät eine tägliche Energieaufnahme von weniger als dem täglichen Energieverbrauch (TEE) des älteren Erwachsenen beinhaltet, wobei die hypokalorische Diät vorzugsweise 25 bis 95 %, vorzugsweise 60 bis 90 %, bevorzugter 70 bis 90 % des TEE des adipösen oder übergewichtigen Erwachsenen liefert.

6. Produkt zur Anwendung nach einem der Ansprüche 4 oder 5, wobei das Trainingsprogramm ein Krafttraining beinhaltet.

7. Produkt zur Anwendung nach einem der voranstehenden Ansprüche, wobei die Portion als Einzelgabe verabreicht wird, die innerhalb von 15 Minuten, vorzugsweise innerhalb von 10 Minuten, noch bevorzugter innerhalb von 5 Minuten, zu konsumieren ist.

8. Produkt zur Anwendung nach einem der voranstehenden Ansprüche, wobei mindestens 13 Gew.-%, vorzugsweise zwischen 13 und 20 Gew.-% der gesamten proteinhaltigen Substanz Leucin ist.

9. Produkt zur Anwendung nach einem der voranstehenden Ansprüche, wobei die Zusammensetzung pro Portion mindestens 0,5 Gramm mindestens einer Verbindung umfasst, die aus der Gruppe ausgewählt ist, die aus freiem Leucin, Salzen von freiem Leucin oder Leucin-Metaboliten oder Salzen von Leucin-Metaboliten besteht, wobei der Metabolit vorzugsweise aus der Gruppe ausgewählt ist, die aus β-Hydroxy-β-methylbutyrat, β-Hydroxy-β-methylbutyrat-freier Säure, Calcium-β-hydroxy-β-methylbutyrat, Hydroxyisocapronsäure und Ketoisocapronsäure besteht, und wobei das freie Leucin, Salze davon oder seine Metaboliten oder das Isoleucin oder Valin vorzugsweise eingekapselt sind.

10. Produkt zur Anwendung nach einem der voranstehenden Ansprüche, wobei die Zusammensetzung weiter mindestens 20 Energieprozent Kohlenhydrate und/oder Fett umfasst.

11. Produkt zur Anwendung nach Anspruch 10, wobei die Kohlenhydrate mindestens 10 Gew.-%, basierend auf der gesamten Kohlenhydratmasse, eines Kohlenhydrats mit einem glykämischen Index unter 70 umfassen.

12. Produkt zur Anwendung nach Anspruch 10, wobei das Fett mindestens 30 Gew.-% ungesättigte Fettsäuren und mindestens 15 Gew.-% mehrfach ungesättigte Säuren des Gesamtfetts in der Zusammensetzung umfasst.

13. Produkt zur Anwendung nach einem der voranstehenden Ansprüche, wobei die proteinhaltige Substanz mindestens 60 Gew.-% Säugetiermolke, vorzugsweise Rindermolke, umfasst.

14. Produkt zur Anwendung nach einem der voranstehenden Ansprüche, wobei die Portion zwischen 0,5 und 6 g Ballaststoffe umfasst, wobei die Ballaststoffe mindestens eine Faser umfassen, die aus der Gruppe ausgewählt ist, die aus Galactooligosacchariden (GOS), Fructooligosacchariden (FOS), Inulin und Pektinoligosacchariden besteht, vorzugsweise mindestens eine Faser, die aus der Gruppe ausgewählt ist, die aus GOS und FOS besteht, besonders bevorzugt eine Mischung aus GOS und FOS.

## Revendications

1. - Produit nutritionnel pour une utilisation dans :
a) la diminution des taux d'insuline à jeun ; ou
b) l'augmentation de la sensibilité à l'insuline ; ou
c) la diminution de la résistance à l'insuline,
chez des adultes obèses ou en surpoids âgés d'au moins 45 ans,
ledit produit comprenant par portion entre 50 et 300 kcal ; entre 10 g et 35 g de matière protéique ; et au moins 2,5 microgrammes de vitamine **D.**

2. - Produit pour l'utilisation selon la revendication **1,** dans lequel l'adulte souffre d'une maladie **ou d'un** trouble choisi dans le groupe consistant en le prédiabète de type 2 ou le diabète de type **2.**

3. - Produit pour l'utilisation selon l'une quelconque des revendications précédentes, dans lequel le traitement comprend une dose quotidienne de 1 à 4 portions.

4. - Produit pour l'utilisation selon l'une quelconque des revendications précédentes, dans lequel les adultes en surpoids ou obèses participent à un programme de perte de poids, le programme de perte de poids comprenant de préférence un régime hypocalorique en association avec un programme d'exercices.

5. - Produit pour l'utilisation selon la revendication 4, dans lequel ledit régime hypocalorique implique un apport énergétique journalier inférieur à la dépense énergétique journalière (DEJ) de l'adulte âgé, le régime hypocalorique de préférence fournissant 25 à 95 %, de préférence 60 à 90 %, de façon davantage préférée 70 à 90 % de la DEJ de l'adulte obèse ou en surpoids.

6. - Produit pour l'utilisation selon l'une quelconque des revendications 4 ou 5, dans lequel ledit programme d'exercices implique un entraînement à des exercices de résistance.

7. - Produit pour l'utilisation selon l'une quelconque des revendications précédentes, dans lequel la portion est administrée en une seule prise à consommer dans les 15 minutes, de façon davantage préférée dans les 10 minutes, de façon encore plus préférée dans les 5 minutes.

8. - Produit pour l'utilisation selon l'une quelconque des revendications précédentes, dans lequel au moins 13 % en poids, de préférence entre 13 et 20 % en poids de la matière protéique totale est de la leucine.

9. - Produit pour l'utilisation selon l'une quelconque des revendications précédentes, dans lequel la composition comprend par portion au moins 0,5 gramme d'au moins un composé choisi dans le groupe consistant en la leucine libre, les sels de leucine libre ou les métabolites de la leucine ou les sels de métabolites de la leucine, le métabolite étant de préférence choisi dans le groupe consistant en le β-hydroxy-β-méthylbutyrate, le β-hydroxy-β-méthylbutyrate acide libre, le calcium-β-hydroxy-β-méthylbutyrate, l'acide hydroxyl iso caproïque et l'acide cétoisocaproïque, et la leucine libre, les sels de celle-ci ou ses métabolites ou l'isoleucine ou la valine étant de préférence encapsulés.

10. - Produit pour l'utilisation selon l'une quelconque des revendications précédentes, dans lequel la composition comprend en outre au moins 20 % en énergie de glucides et/ou de matières grasses.

11. - Produit pour l'utilisation selon la revendication 10, dans lequel les glucides comprennent au moins 10 % en poids, sur la base de la matière glucidique totale, d'un glucide ayant un indice glycémique inférieur à 70.

12. - Produit pour l'utilisation selon la revendication 10, dans lequel la matière grasse comprend au moins 30 % en poids d'acides gras insaturés et au moins 15 % en poids d'acides polyinsaturés de la graisse totale dans la composition.

13. - Produit pour l'utilisation selon l'une quelconque des revendications précédentes, dans lequel la matière protéique comprend au moins 60 % en poids de lactosérum de mammifère, de préférence de lactosérum bovin.

14. - Produit pour l'utilisation selon l'une quelconque des revendications précédentes, dans lequel la portion comprend entre 0,5 et 6 g de fibres alimentaires, les fibres alimentaires comprenant au moins une fibre choisie dans le groupe consistant en les galacto-oligosaccharides (GOS), les fructooligosaccharides (FOS), les oligosaccharides d'inuline et de pectine, de préférence au moins une fibre choisie dans le groupe consistant en les GOS et les FOS, de façon davantage préférée un mélange de GOS et de FOS.
